# EUROPEAN PATENT SPECIFICATION

(11) **EP 3 730 610 B1**
(45) Date of publication and mention of the grant of the patent: **13.04.2022**
(21) Application number: 19171002.9
(22) Date of filing: 25.04.2019
(51) Int. Cl.: C12N 9/22, C12N 15/62

(54) **MODIFIED CAS9 SYSTEM AND ITS USE FOR IMPROVED GENE EDITING**
MODIFIZIERTES CAS9-SYSTEM UND DESSEN VERWENDUNG ZUR VERBESSERTEN GENEDITIERUNG
SYSTÈME CAS9 MODIFIÉ ET SON UTILISATION POUR L'AMÉLIORATION DE L'ÉDITION GÉNIQUE

(43) Date of publication of application: 28.10.2020
(73) Proprietor: Albert-Ludwigs-Universität Freiburg, 79098 Freiburg (DE)
(72) Inventor: Mussolino, Claudio, 79115 Freiburg (DE); Cathomen, Toni, 79106 Freiburg (DE); Cornu, Tatjana, 79106 Freiburg (DE); Carusillo, Antonio, 79098 Freiburg (DE)
(74) Representative: Lederer & Keller Patentanwälte Partnerschaft mbB

(56) References cited:
- WO-A1-2016/054326
- WO-A1-2017/142923
- WO-A1-2017/180694
- SIMIN SHAO ET AL: "Enhancing CRISPR/Cas9-mediated homology-directed repair in mammalian cells by expressing Saccharomyces cerevisiae Rad52", INTERNATIONAL JOURNAL OF BIOCHEMISTRY AND CELL BIOLOGY, vol. 92, 18 September 2017 (2017-09-18), pages 43-52, XP055627157, GB ISSN: 1357-2725, DOI: 10.1016/j.biocel.2017.09.012
- LING WANG ET AL: "Enhancing Targeted Genomic DNA Editing in Chicken Cells Using the CRISPR/Cas9 System", PLOS ONE, vol. 12, no. 1, 9 January 2017 (2017-01-09), page e0169768, XP055385966, DOI: 10.1371/journal.pone.0169768

### SEQUENCE LISTING

<110> Albert-Ludwigs-Universität Freiburg
<120> Modified Cas9 system and its use for improved gene editing
<130> PEP03137UNI
<160> 13
<170> PatentIn version 3.5
<210> 1
   <211> 13
   <212> PRT
   <213> artificial sequence
<220>
   <223> linker
<400> 1
   Ser Ala Ala Gly Gly Gly Gly Ser Gly Pro Arg Ser Gly
   1               5                   10
<210> 2
   <211> 14
   <212> PRT
   <213> artificial sequence
<220>
   <223> linker
<400> 2
   Thr Val Asp Ser Ala Ala Gly Gly Gly Gly Ser Gly Pro Ser
   1               5                   10
<210> 3
   <211> 1367
   <212> PRT
   <213> artificial sequence
<220>
   <223> Cas9 nuclease
<400> 3
   Asp Lys Lys Tyr Ser Ile Gly Leu Asp Ile Gly Thr Asn Ser Val Gly
   1               5                   10                  15
   Trp Ala Val Ile Thr Asp Glu Tyr Lys Val Pro Ser Lys Lys Phe Lys
               20                  25                  30
   Val Leu Gly Asn Thr Asp Arg His Ser Ile Lys Lys Asn Leu Ile Gly
           35                  40                  45
   Ala Leu Leu Phe Asp Ser Gly Glu Thr Ala Glu Ala Thr Arg Leu Lys
       50                  55                  60
   Arg Thr Ala Arg Arg Arg Tyr Thr Arg Arg Lys Asn Arg Ile Cys Tyr
   65                  70                  75                  80
   Leu Gln Glu Ile Phe Ser Asn Glu Met Ala Lys Val Asp Asp Ser Phe
                   85                  90                  95
   Phe His Arg Leu Glu Glu Ser Phe Leu Val Glu Glu Asp Lys Lys His
               100                 105                 110
   Glu Arg His Pro Ile Phe Gly Asn Ile Val Asp Glu Val Ala Tyr His
           115                 120                 125
   Glu Lys Tyr Pro Thr Ile Tyr His Leu Arg Lys Lys Leu Val Asp Ser
       130                 135                 140
   Thr Asp Lys Ala Asp Leu Arg Leu Ile Tyr Leu Ala Leu Ala His Met
   145                 150                 155                 160
   Ile Lys Phe Arg Gly His Phe Leu Ile Glu Gly Asp Leu Asn Pro Asp
                   165                 170                 175
   Asn Ser Asp Val Asp Lys Leu Phe Ile Gln Leu Val Gln Thr Tyr Asn
               180                 185                 190
   Gln Leu Phe Glu Glu Asn Pro Ile Asn Ala Ser Gly Val Asp Ala Lys
           195                 200                 205
   Ala Ile Leu Ser Ala Arg Leu Ser Lys Ser Arg Arg Leu Glu Asn Leu
       210                 215                 220
   Ile Ala Gln Leu Pro Gly Glu Lys Lys Asn Gly Leu Phe Gly Asn Leu
   225                 230                 235                 240
   Ile Ala Leu Ser Leu Gly Leu Thr Pro Asn Phe Lys Ser Asn Phe Asp
                   245                 250                 255
   Leu Ala Glu Asp Ala Lys Leu Gln Leu Ser Lys Asp Thr Tyr Asp Asp
               260                 265                 270
   Asp Leu Asp Asn Leu Leu Ala Gln Ile Gly Asp Gln Tyr Ala Asp Leu
           275                 280                 285
   Phe Leu Ala Ala Lys Asn Leu Ser Asp Ala Ile Leu Leu Ser Asp Ile
       290                 295                 300
   Leu Arg Val Asn Thr Glu Ile Thr Lys Ala Pro Leu Ser Ala Ser Met
   305                 310                 315                 320
   Ile Lys Arg Tyr Asp Glu His His Gln Asp Leu Thr Leu Leu Lys Ala
                   325                 330                 335
   Leu Val Arg Gln Gln Leu Pro Glu Lys Tyr Lys Glu Ile Phe Phe Asp
               340                 345                 350
   Gln Ser Lys Asn Gly Tyr Ala Gly Tyr Ile Asp Gly Gly Ala Ser Gln
           355                 360                 365
   Glu Glu Phe Tyr Lys Phe Ile Lys Pro Ile Leu Glu Lys Met Asp Gly
       370                 375                 380
   Thr Glu Glu Leu Leu Val Lys Leu Asn Arg Glu Asp Leu Leu Arg Lys
   385                 390                 395                 400
   Gln Arg Thr Phe Asp Asn Gly Ser Ile Pro His Gln Ile His Leu Gly
                   405                 410                 415
   Glu Leu His Ala Ile Leu Arg Arg Gln Glu Asp Phe Tyr Pro Phe Leu
               420                 425                 430
   Lys Asp Asn Arg Glu Lys Ile Glu Lys Ile Leu Thr Phe Arg Ile Pro
           435                 440                 445
   Tyr Tyr Val Gly Pro Leu Ala Arg Gly Asn Ser Arg Phe Ala Trp Met
       450                 455                 460
   Thr Arg Lys Ser Glu Glu Thr Ile Thr Pro Trp Asn Phe Glu Glu Val
   465                 470                 475                 480
   Val Asp Lys Gly Ala Ser Ala Gln Ser Phe Ile Glu Arg Met Thr Asn
                   485                 490                 495
   Phe Asp Lys Asn Leu Pro Asn Glu Lys Val Leu Pro Lys His Ser Leu
               500                 505                 510
   Leu Tyr Glu Tyr Phe Thr Val Tyr Asn Glu Leu Thr Lys Val Lys Tyr
           515                 520                 525
   Val Thr Glu Gly Met Arg Lys Pro Ala Phe Leu Ser Gly Glu Gln Lys
       530                 535                 540
   Lys Ala Ile Val Asp Leu Leu Phe Lys Thr Asn Arg Lys Val Thr Val
   545                 550                 555                 560
   Lys Gln Leu Lys Glu Asp Tyr Phe Lys Lys Ile Glu Cys Phe Asp Ser
   565 570 575
   Val Glu Ile Ser Gly Val Glu Asp Arg Phe Asn Ala Ser Leu Gly Thr
   580 585 590
   Tyr His Asp Leu Leu Lys Ile Ile Lys Asp Lys Asp Phe Leu Asp Asn
   595 600 605
   Glu Glu Asn Glu Asp Ile Leu Glu Asp Ile Val Leu Thr Leu Thr Leu
   610 615 620
   Phe Glu Asp Arg Glu Met Ile Glu Glu Arg Leu Lys Thr Tyr Ala His
   625 630 635 640
   Leu Phe Asp Asp Lys Val Met Lys Gln Leu Lys Arg Arg Arg Tyr Thr
   645 650 655
   Gly Trp Gly Arg Leu Ser Arg Lys Leu Ile Asn Gly Ile Arg Asp Lys
   660 665 670
   Gln Ser Gly Lys Thr Ile Leu Asp Phe Leu Lys Ser Asp Gly Phe Ala
   675 680 685
   Asn Arg Asn Phe Met Gln Leu Ile His Asp Asp Ser Leu Thr Phe Lys
   690 695 700
   Glu Asp Ile Gln Lys Ala Gln Val Ser Gly Gln Gly Asp Ser Leu His
   705 710 715 720
   Glu His Ile Ala Asn Leu Ala Gly Ser Pro Ala Ile Lys Lys Gly Ile
   725 730 735
   Leu Gln Thr Val Lys Val Val Asp Glu Leu Val Lys Val Met Gly Arg
   740 745 750
   His Lys Pro Glu Asn Ile Val Ile Glu Met Ala Arg Glu Asn Gln Thr
   755 760 765
   Thr Gln Lys Gly Gln Lys Asn Ser Arg Glu Arg Met Lys Arg Ile Glu
   770 775 780
   Glu Gly Ile Lys Glu Leu Gly Ser Gln Ile Leu Lys Glu His Pro Val
   785 790 795 800
   Glu Asn Thr Gln Leu Gln Asn Glu Lys Leu Tyr Leu Tyr Tyr Leu Gln
   805 810 815
   Asn Gly Arg Asp Met Tyr Val Asp Gln Glu Leu Asp Ile Asn Arg Leu
               820                 825                 830
   Ser Asp Tyr Asp Val Asp His Ile Val Pro Gln Ser Phe Leu Lys Asp
           835                 840                 845
   Asp Ser Ile Asp Asn Lys Val Leu Thr Arg Ser Asp Lys Asn Arg Gly
       850                 855                 860
   Lys Ser Asp Asn Val Pro Ser Glu Glu Val Val Lys Lys Met Lys Asn
   865                 870                 875                 880
   Tyr Trp Arg Gln Leu Leu Asn Ala Lys Leu Ile Thr Gln Arg Lys Phe
                   885                 890                 895
   Asp Asn Leu Thr Lys Ala Glu Arg Gly Gly Leu Ser Glu Leu Asp Lys
               900                 905                 910
   Ala Gly Phe Ile Lys Arg Gln Leu Val Glu Thr Arg Gln Ile Thr Lys
           915                 920                 925
   His Val Ala Gln Ile Leu Asp Ser Arg Met Asn Thr Lys Tyr Asp Glu
       930                 935                 940
   Asn Asp Lys Leu Ile Arg Glu Val Lys Val Ile Thr Leu Lys Ser Lys
   945                 950                 955                 960
   Leu Val Ser Asp Phe Arg Lys Asp Phe Gln Phe Tyr Lys Val Arg Glu
                   965                 970                 975
   Ile Asn Asn Tyr His His Ala His Asp Ala Tyr Leu Asn Ala Val Val
               980                 985                 990
   Gly Thr Ala Leu Ile Lys Lys Tyr  Pro Lys Leu Glu Ser  Glu Phe Val
           995                 1000                 1005
   Tyr Gly  Asp Tyr Lys Val Tyr  Asp Val Arg Lys Met  Ile Ala Lys
       1010                 1015                 1020
   Ser Glu  Gln Glu Ile Gly Lys  Ala Thr Ala Lys Tyr  Phe Phe Tyr
       1025                 1030                 1035
   Ser Asn  Ile Met Asn Phe Phe  Lys Thr Glu Ile Thr  Leu Ala Asn
       1040                 1045                 1050
   Gly Glu  Ile Arg Lys Arg Pro  Leu Ile Glu Thr Asn  Gly Glu Thr
   1055 1060 1065
   Gly Glu Ile Val Trp Asp Lys Gly Arg Asp Phe Ala Thr Val Arg
   1070 1075 1080
   Lys Val Leu Ser Met Pro Gln Val Asn Ile Val Lys Lys Thr Glu
   1085 1090 1095
   Val Gln Thr Gly Gly Phe Ser Lys Glu Ser Ile Leu Pro Lys Arg
   1100 1105 1110
   Asn Ser Asp Lys Leu Ile Ala Arg Lys Lys Asp Trp Asp Pro Lys
   1115 1120 1125
   Lys Tyr Gly Gly Phe Asp Ser Pro Thr Val Ala Tyr Ser Val Leu
   1130 1135 1140
   Val Val Ala Lys Val Glu Lys Gly Lys Ser Lys Lys Leu Lys Ser
   1145 1150 1155
   Val Lys Glu Leu Leu Gly Ile Thr Ile Met Glu Arg Ser Ser Phe
   1160 1165 1170
   Glu Lys Asn Pro Ile Asp Phe Leu Glu Ala Lys Gly Tyr Lys Glu
   1175 1180 1185
   Val Lys Lys Asp Leu Ile Ile Lys Leu Pro Lys Tyr Ser Leu Phe
   1190 1195 1200
   Glu Leu Glu Asn Gly Arg Lys Arg Met Leu Ala Ser Ala Gly Glu
   1205 1210 1215
   Leu Gln Lys Gly Asn Glu Leu Ala Leu Pro Ser Lys Tyr Val Asn
   1220 1225 1230
   Phe Leu Tyr Leu Ala Ser His Tyr Glu Lys Leu Lys Gly Ser Pro
   1235 1240 1245
   Glu Asp Asn Glu Gln Lys Gln Leu Phe Val Glu Gln His Lys His
   1250 1255 1260
   Tyr Leu Asp Glu Ile Ile Glu Gln Ile Ser Glu Phe Ser Lys Arg
   1265 1270 1275
   Val Ile Leu Ala Asp Ala Asn Leu Asp Lys Val Leu Ser Ala Tyr
   1280 1285 1290
   Asn Lys  His Arg Asp Lys Pro  Ile Arg Glu Gln Ala  Glu Asn Ile
       1295                 1300                 1305
   Ile His  Leu Phe Thr Leu Thr  Asn Leu Gly Ala Pro  Ala Ala Phe
       1310                 1315                 1320
   Lys Tyr  Phe Asp Thr Thr Ile  Asp Arg Lys Arg Tyr  Thr Ser Thr
       1325                 1330                 1335
   Lys Glu  Val Leu Asp Ala Thr  Leu Ile His Gln Ser  Ile Thr Gly
       1340                 1345                 1350
   Leu Tyr  Glu Thr Arg Ile Asp  Leu Ser Gln Leu Gly  Gly Asp
       1355                 1360                 1365
<210> 4
   <211> 339
   <212> PRT
   <213> artificial sequence
<220>
   <223> RAD51
<400> 4
<210> 5
   <211> 418
   <212> PRT
   <213> artificial sequence
<220>
   <223> RAD52
<400> 5
   Met Ser Gly Thr Glu Glu Ala Ile Leu Gly Gly Arg Asp Ser His Pro
   1               5                   10                  15
   Ala Ala Gly Gly Gly Ser Val Leu Cys Phe Gly Gln Cys Gln Tyr Thr
               20                  25                  30
   Ala Glu Glu Tyr Gln Ala Ile Gln Lys Ala Leu Arg Gln Arg Leu Gly
           35                  40                  45
   Pro Glu Tyr Ile Ser Ser Arg Met Ala Gly Gly Gly Gln Lys Val Cys
       50                  55                  60
   Tyr Ile Glu Gly His Arg Val Ile Asn Leu Ala Asn Glu Met Phe Gly
   65                  70                  75                  80
   Tyr Asn Gly Trp Ala His Ser Ile Thr Gln Gln Asn Val Asp Phe Val
                   85                  90                  95
   Asp Leu Asn Asn Gly Lys Phe Tyr Val Gly Val Cys Ala Phe Val Arg
               100                 105                 110
   Val Gln Leu Lys Asp Gly Ser Tyr His Glu Asp Val Gly Tyr Gly Val
           115                 120                 125
   Ser Glu Gly Leu Lys Ser Lys Ala Leu Ser Leu Glu Lys Ala Arg Lys
       130                 135                 140
   Glu Ala Val Thr Asp Gly Leu Lys Arg Ala Leu Arg Ser Phe Gly Asn
   145                 150                 155                 160
   Ala Leu Gly Asn Cys Ile Leu Asp Lys Asp Tyr Leu Arg Ser Leu Asn
                   165                 170                 175
   Lys Leu Pro Arg Gln Leu Pro Leu Glu Val Asp Leu Thr Lys Ala Lys
               180                 185                 190
   Arg Gln Asp Leu Glu Pro Ser Val Glu Glu Ala Arg Tyr Asn Ser Cys
           195                 200                 205
   Arg Pro Asn Met Ala Leu Gly His Pro Gln Leu Gln Gln Val Thr Ser
       210                 215                 220
   Pro Ser Arg Pro Ser His Ala Val Ile Pro Ala Asp Gln Asp Cys Ser
   225                 230                 235                 240
   Ser Arg Ser Leu Ser Ser Ser Ala Val Glu Ser Glu Ala Thr His Gln
                   245                 250                 255
   Arg Lys Leu Arg Gln Lys Gln Leu Gln Gln Gln Phe Arg Glu Arg Met
               260                 265                 270
   Glu Lys Gln Gln Val Arg Val Ser Thr Pro Ser Ala Glu Lys Ser Glu
           275                 280                 285
   Ala Ala Pro Pro Ala Pro Pro Val Thr His Ser Thr Pro Val Thr Val
       290                 295                 300
   Ser Glu Pro Leu Leu Glu Lys Asp Phe Leu Ala Gly Val Thr Gln Glu
   305                 310                 315                 320
   Leu Ile Lys Thr Leu Glu Asp Asn Ser Glu Lys Trp Ala Val Thr Pro
                   325                 330                 335
   Asp Ala Gly Asp Gly Val Val Lys Pro Ser Ser Arg Ala Asp Pro Ala
               340                 345                 350
   Gln Thr Ser Asp Thr Leu Ala Leu Asn Asn Gln Met Val Thr Gln Asn
           355                 360                 365
   Arg Thr Pro His Ser Val Cys His Gln Lys Pro Gln Ala Lys Ser Gly
       370                 375                 380
   Ser Trp Asp Leu Gln Thr Tyr Ser Ala Asp Gln Arg Thr Thr Gly Asn
   385                 390                 395                 400
   Trp Glu Ser His Arg Lys Ser Gln Asp Met Lys Lys Arg Lys Tyr Asp
                   405                 410                 415
   Pro Ser
<210> 6
   <211> 747
   <212> PRT
   <213> artificial sequence
<220>
   <223> RAD54
<400> 6
<210> 7
   <211> 708
   <212> PRT
   <213> artificial sequence
<220>
   <223> MRE11
<400> 7
<210> 8
   <211> 1186
   <212> PRT
   <213> artificial sequence
<220>
   <223> PALB2
<400> 8
<210> 9
   <211> 1471
   <212> PRT
   <213> artificial sequence
<220>
   <223> FANCD2
<400> 9
<210> 10
   <211> 846
   <212> PRT
   <213> artificial sequence
<220>
   <223> EXO1
<400> 10
<210> 11
   <211> 897
   <212> PRT
   <213> artificial sequence
<220>
   <223> CtIP
<400> 11
<210> 12
   <211> 18
   <212> PRT
   <213> artificial sequence
<220>
   <223> nuclear localisation signal
<400> 12
   Lys Arg Thr Ala Asp Gly Ser Glu Phe Glu Ser Pro Lys Lys Lys Arg
   1               5                   10                  15
   Lys Val
<210> 13
   <211> 9
   <212> PRT
   <213> artificial sequence
<220>
   <223> hemaglutination tag
<400> 13
   Tyr Pro Tyr Asp Val Pro Asp Tyr Ala
   1               5

## Description

### Background of the invention

Gene therapy relies on the use of genes to cure genetic defects identified in patients. In general, the approach of gene therapy can be used to reconstitute a function which is missing or lost in the diseased cells of the patient. In the last decades, gene therapy has been widely used to treat primary immunodeficiencies caused by mutations that inactivate key genes in the hematopoietic system. A typical gene therapy approach in this case is to use natural viral vectors, modified in a way that viral genetic elements are removed and substituted with an expression cassette encoding for the gene which is missing in the patient's cells. Hematopoietic cells derived from the patient can be then isolated and transduced with the recombinant vector; this results in the integration of the viral genome into the host genome leading to the expression of the gene harbored by the virus. As a consequence, the expression of the gene missing in patient cells is reconstituted from the new gene product delivered by the virus. The modified cells can be used as a gene therapy product and transplanted back to the original patient as a therapy. Even though this approach has shown successful for the treatment of multiple immunodeficiencies, it relies on the random mechanism by which the viral genome is integrated in the host cell. This poses serious safety concerns as integration close to oncogenes or oncosuppressors might lead to uncontrolled proliferation of the modified cells and, even if rare, such events can lead on the long run to the occurrence of cancer.

The correction of DNA breaks can occur either by homology-directed repair (HDR) which is typically much less efficient than the non-homologous end-joining (NHEJ) repair. Because of the low efficiency of HDR-based targeted genomic modification it is often far below the desired clinically relevant frequencies. The therapeutic potential of precise genome editing remains therefore a largely unexplored opportunity. Current strategies to increase the low rate of HDR-based gene editing rely mostly on the use of small molecule drugs to either block the cells in the S/G2 cell cycle phase, when the HDR pathway is most active or to inhibit the NHEJ. Typically such small molecules have a broad impact on cellular physiology and while they may be effective in some systems (mostly *in vitro* systems) their implication for clinically oriented studies might be undermined by safety concerns and an unfavorable risk/benefit ratio.

### Object of the invention

In the last decade, methods to precisely modify patient's own cells without using randomly integrating viral vectors have become more and more attractive. Precise modification of a genome includes both the integration of a gene expression cassette in a very precise location of the host genome previously validated to be safe when modified, the so-called "safe harbors", and the precise reversion of a disease causing mutation to the normal sequence. Both cases rely on delivering into the cells a fragment of exogenous DNA (the so-called donor DNA) homologous to the target site and containing either the expression cassette to be integrated or the normal sequence to correct the underlying mutation.

In order to allow the transfer of the genetic information from the exogenous DNA to the precise location of the genome to be modified, the cells use the mechanism of Homologous Recombination (HR) also called Homology Directed Repair (HDR). Typically, HR is a very rare event in mammalian cells but this frequency can be increased to a relevant extent if a DNA double strand break (DSB) occurs at the target site and at the same time a homologous donor DNA is provided. Thereby in the last 20 years, huge efforts have been devoted to characterize and improve platforms to create DSBs in specific genomic locations and this has resulted in the introduction of designer nucleases (DN) as zinc finger nucleases (ZFNs), transcription activator-like effector nucleases (TALENs) and CRISPR-Cas nucleases.

Among these, Designer Nucleases (DNs) based on the CRISPR-Cas systems are to date among the most widely used as a consequence of their versatility and efficiency. However, when a DSB occurs, mammalian cells rely mostly on the non-homologous end-joining (NHEJ) DNA repair pathway to repair the break and survive. This pathway is error-prone and does not lead to the incorporation of the genetic information from the exogenous DNA to DSB site. Even when using highly efficient DNs, HDR-mediated gene editing is still extremely inefficient as NHEJ-mediated correction of a DSB is preferred leading to small mutations at the site of the break.

This unbalance between NHEJ and HR strongly hampers the applicability of precise genome editing as novel therapeutic option for human disorders. Indeed, the frequency of precise genome editing events in hematopoietic stem cells, the most relevant cell type in the field of gene therapy of primary immunodeficiency, is highly variable and far below the desired threshold which would be significant for achieving therapeutic benefit in patients. To further increase the low rate of HR-based gene editing, several strategies have been adopted which typically use chemical compounds to change the physiological balance between NHEJ and HDR in favor of the latter. However, even though these strategies have shown promising in some cellular systems, their applicability in clinically relevant settings poses concerns due to the potential side effects related to their use. Therefore, there is a need to develop a novel platform to render HR-mediated genome editing more efficient and potentially applicable into clinics.

Some years ago, a unique pathway has been discovered which uses bacteria and archea to defend themselves from cellular invaders like plasmids or bacteriophages. Such defense mechanism has been designated as Clustered Regulatory Interspaced Short Palindromic Repeats (CRISPR) along with the CRISPR associated proteins which are shortly designated as Cas proteins. Meanwhile three different types of CRISPR-Cas systems have been identified in bacteria and archea, namely Type I, Type II and Type III. The Type II CRISPR system has been most commonly adapted for genome editing due to its simplicity requiring just one Cas protein, Cas9 and of course RNA components. The RNA components are required for bringing the nuclease to the desired site at the DNA sequence to be edited.

The HDR-CRISPR complex as described herein can be easily delivered to clinically relevant primary human cells in the form of mRNA/gRNA or as protein/gRNA. It offers therefore a strategy for improving HDR-based genome editing in therapeutically relevant cell types.

Cas9 wild type contains two nuclease domains designated as RuvC and HNH which each cut a different strand of the DNA. The HNH domain nicks the DNA strand that is complementary to the crRNA and the RuvC-like domain nicks the strand that is not complementary to the crRNA. Cas9 cleaves the DNA three base pairs upstream of the protospacer adjacent motif (PAM), resulting in a blunt-end cleavage of DNA. Cleaving the DNA is deleterious to the invading plasmid or virus, resulting in degradation and protection against these invaders. A double-strand break is highly efficient in the degradation of foreign DNA since the Cas9 induced double-strand breaks can be repaired by non-homologous end joining (NHEJ) which may easily result in insertions and/or deletions (indels). For genome editing the NHEJ is, however, disadvantageous.

Charpentier et al., Nature Communications (2018) 9:1133 [DOI: 10.1038/s41467-018-03475-7] describe the fusion of CtIP to Cas9 in order to enhance the transgene integration by homology-dependent repair (HDR). WO 2017/142923 A1 discloses the fusion of CtIP or an HDR factor to Cas9. Z

### Field of invention

The so-called homology-directed repair (HDR) is for the genetic engineering more valuable since a precise modification of the sequence can be obtained. In order to make the activity of Cas9 more specific the RuvC catalytic domain may be inactivated for example by a single amino acid mutation that inactivates this domain. Such mutation may be for example the mutation D10A. A Cas9 having such a mutation has only the HNH catalytic domain which cuts just one strand of the DNA that is complementary to the sgRNA. Such variant is also designated "nickase Cas9" or "nCas9". In preferred embodiments such nCas9 are used for the homologous directed repair (HDR).

The invention provides a modified Cas9 nuclease comprising a substantial part of a Cas9 nuclease which is required for the biological activity and fused thereto at least one of a functional protein domain of a HDR factor selected from the group consisting of RAD51, RAD52, and/or MRE11, characterized in that it further comprises CtBP interacting protein (CtIP).

In a preferred embodiment of the present invention a modified Cas9 protein is disclosed which is able to instruct the DNA repair machinery of the cell to repair a double strand break (DSB) via the homologous directed repair (HDR) pathway with higher frequency as compared to physiological conditions. In particular, a fusion of an active nuclease, the Cas9 protein derived from *Streptococcus pyogenes* (SpCas9), and a combination of one or multiple factors involved in the HDR pathway for DNA repair is described.

The modified Cas9 complex according to the invention may be used in a system which can be designated shortly as "HDR-CRISPR". In general after the introduction of a double-strand break of the nucleic acid induced by Cas9 which is preferably derived from *Streptococcus pyogenes* the presence of HDR factors directly fused or linked to the Cas9 molecules at the target site drives the cell to repair the break in the DNA by engaging the HDR-mediated DNA repair pathway. One of the advantages of the preferred embodiments of the invention is the broad application since it can be used in all instances where precise DNA changes are required, as for example in order to correct a point mutation in cells derived from the patient. Alternatively, it can be used in order to integrate the expression cassette of a gene of interest in a very specific position of the target genome.

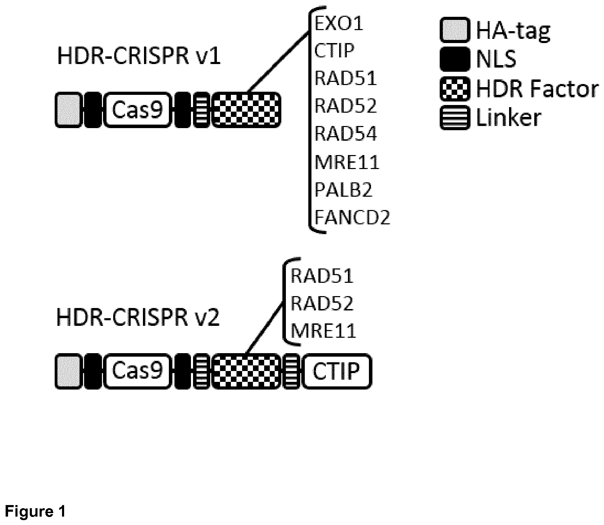

The mutated Cas9 which is one of the essential components of the HDR-CRISPR can be produced by recombinant means. In this case the nucleic acid coding for the Cas9 may be linked directly to genes coding for the HDR-promoting factors and this construct may be inserted into a vector which may have viral or plasmid-derived components. Such a vector may be able to replicate in the host cell and to express the modified Cas9 protein comprising the preferred HDR-promoting factors which may be linked via an amino acid linker having for example the sequence SAAGGGGSGPRSG (SEQ ID NO:1) or an amino acid linker having the sequence TVDSAAGGGGSGPS (SEQ ID NO:2). Such modified Cas9 fusion proteins are typically expressed in a cell line from the plasmid that contains also a promoter which may preferably be derived from CMV (cytomegalovirus) followed by the construct. One example of such a construct can be seen in Figure 1.

It is a preferred embodiment of the present invention to deliver the modified Cas9 structure according to the present invention in the format of a replicable element into the target cells which may be human primary cells since there are several protocols available how such Cas9 proteins can be introduced into the target cells. Of course the RNA components required for the function of the CRISPR-Cas system must also be present in the target cells. Such RNA components can be present either on the same replicating unit or they can alternatively be present in a further vector or several vectors.

In the present invention the modified Cas9 nuclease (SEQ ID NO:3) comprises at least a substantial part of the Cas nuclease which is required for the biological activity. At least one functional part or preferably the whole molecule of a HDR factor is directly fused to the Cas9 via an amino acid linker.

The Cas9 nuclease is in preferred embodiments directly fused via a linker to the HDR factors whereby the linker is expressed from the genetic construct coding therefor. In an alternative embodiment, however, the Cas9 nuclease may be linked to the HDR factor by a chemical linker. Such embodiment may be suitable for certain in vitro modifications of the genome.

The HDR factors are selected from the group consisting of the factors having the designation:
RAD51 (SEQ ID NO:4)
RAD52 (SEQ ID NO:5)
RAD54 (SEQ ID NO:6)
MRE11 (SEQ ID NO:7)
PALB2 (SEQ ID NO:8)
FANCD2 (SEQ ID NO:9)
EXO1 (SEQ ID NO:10).

In the modified Cas9 construct of the invention, Z the HDR factors are selected from the group consisting of RAD51, RAD52 and MRE11.

Another protein which is added to the modified Cas9 construct is CtIP (SEQ ID NO:11). CtIP is added to the construct only in addition to at least one of the HDR factors mentioned above. CtIP acts as a co-factor for MRE11 endonuclease in triggering DNA end resection.

The Cas9 nuclease fragment or the whole enzyme may be derived from different microorganisms like *Streptococcus pyogenes, Streptococcus thermophiles, Listeria monocytogenes, Staphylococcus aureus, Neisseria meningitidis, Campylobacter jejuni* or other bacteria whereby, however, the Cas9 derived from *Streptococcus pyogenes* is preferred.

The construct of the present invention comprises at least one of the HDR factors, RAD51, RAD52 and Mre11, and it additionally contains CtIP. In particularly preferred embodiment the modified Cas9 nuclease according to the invention comprises two and more preferred of those HDR factors.

In a further embodiment the modified Cas9 nuclease construct according to the present invention may comprise other functional parts like a hemagglutination tag and/or a promoter sequence preferably derived from CMV.

A further embodiment of the present invention relates to the nucleic acids coding for the modified Cas9 nuclease constructs of the invention which can be deduced from the amino acid sequence. Of course a suitable codon usage is selected and the required nucleic acid can easily be synthesized. Such nucleic acid sequences may be inserted into a suitable vector to express the construct. Such vectors may be derived from viral origin or from plasmid origin or may contain functional groups derived from such origins.

In another embodiment of the present invention, the Cas9 nuclease constructs of the invention may be used, preferably in the form of a nucleic acid contained within a suitable vector in a method for editing nucleic acid sequence in a homology-directed repair. Such methods may be used in order to treat patients whereby very precise modifications in certain relevant gene areas are required. By the method according to the present invention it is possible to change the sequence of a gene very precisely in order either to remove an undesired mutation or to induce a certain mutation either in a structural gene or in a regulatory gene sequence like a promoter or an activator and the like.

The method of editing genomic sequences encompasses the modified Cas9 construct as described herein which is usually inserted into the target cell via transfection, transduction, electroporation or cell fusion. The vector is designed in order to allow the replication of the vector in a target cell and the expression of the modified Cas9 complex as described herein. The required RNA sequences may also be encoded on the same vector or alternatively on another vector. It depends on the specific requirements whether all genetic elements required for editing the sequence are contained in one vector or are separated on several, preferably two or not more than three vectors. The use of a vector comprising all required elements is preferred.

The RNA sequences are in a preferred embodiment not further modified. Preferably the gRNA is used which is required for the CRISPR Cas genome edition at the desired position of the genome.

To overcome the problem of low efficiency in HR-mediated genome editing and create a platform readily translatable to clinically oriented applications, HDR-CRISPR was developed. The present invention encompasses the physical fusion between an active Cas9 nuclease with one or multiple proteins or protein domains, involved in promoting the repair of a DSB through homologous recombination. In an alternative it is, however, also possible to link the HDR factors to the Cas9 nuclease by chemical linking methods. Linkers having functional ends which can react with the HDR factors on the one hand and with the Cas9 molecule on the other hand are known to the person skilled in the art and can be used successfully. Such linking may be advantageous when *in vitro* steps like manipulation of gene sequences in cell cultures are performed.

It is assumed that by increasing the concentration of factors that promote the repair of a DSB via HR precisely at the site of double strand break an increased frequency of HR-mediated genome editing is achieved. The invention is applicable in any gene editing strategy that relies on homologous recombination for success. Moreover, the use of the platform in the form of *in vitro* transcribed mRNA or recombinant protein allows delivery directly in clinically relevant primary human cells without toxicity concerns as these delivery methods are relatively safe, well-established and used already in pre-clinical settings.

Preferred embodiments of the present invention are described in the experiments and the Figures.

The ability to precisely correct the genome of a cell harboring a certain disease-causing mutation is still an unmet need in particular in patient-derived primary cells. To contribute in overcoming this problem, the HDR-CRISPR system has been developed. This consists of a fusion between the preferably used Cas9 nuclease from *Streptococcus pyogenes* (SpCas9), with single or multiple protein or protein domains involved in the mechanism of homologous directed repair (HDR). It is assumed that the presence of HDR-promoting proteins or protein domains at the site of a double strand break introduced by the Cas9 nuclease, would drive the repair of the break via the HDR pathway.

Two versions of the HDR-CRISPR were generated which are shown in **Figure 1****.** Both constructs consist of the SpCas9 embedded with two nuclear localization signals (NLS) (SEQ ID NO:12) and a hemagglutinin (HA)-tag (SEQ ID NO:13). In the version 1 (HDR-CRISPR v1), the SpCas9 is directly fused to single proteins or protein domains involved in HDR-mediated DNA repair. The construct shown on top of Figure1 comprises therefore the following components: a hemagglutination tag, a nuclear localization signal, Cas9, a nuclear localization signal, a linker and the HDR factors which may be selected from EXO1, CTIP, RAD51, RAD52, RAD54, MRE11, PALB2 and FANCD2.

The version 2 (HDR-CRISPR v2), includes a fusion of the SpCas9 and CTIP, which has been previously shown to increase the frequency of HDR-mediated repair of DSBs, together with an additional HDR-promoting protein or protein domain. In this construct CTIP which has previously been shown to increase the frequency of HDR-mediated repair of DSB is linked to the HDR factors RAD51, RAD52 and MRE11 whereby on each side of the HDR factors a linker is used.

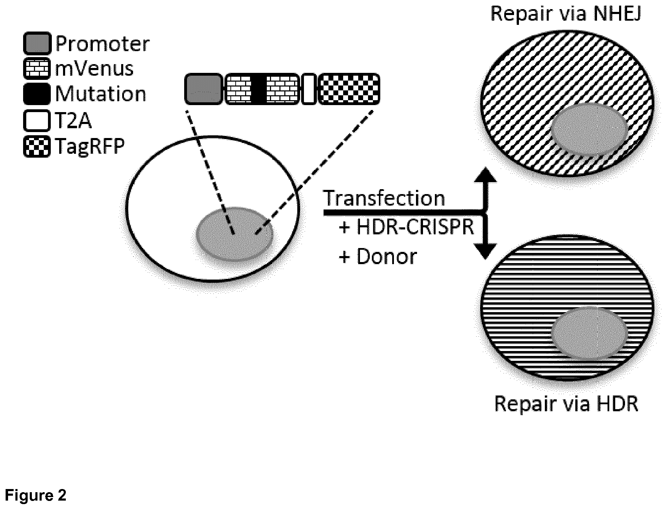

In order to test the ability of our HDR-CRISPR systems to promote the repair of a double strand break via HDR, a traffic light reporter (TLR) system previously described was used (**Figure 2**). In brief, the TLR construct integrated in the cellular genome of the reporter cells (TLR-cells) consists of a mutated green fluorescent protein (mVenus) fused to an out of frame red fluorescent protein (TagRFP) via a T2A self-cleaving peptide. Since mVenus is mutated and the TagRFP is out of frame, the cells harboring the reporter are neither green nor red.

Upon introduction of a DSB by the Cas9 at the site of the mutation in the mVenus sequence, repair via non-homologous end-joining (NHEJ) results in the occurrence of small insertion/deletion (indel) mutations, one third of which would restore the reading frame of the TagRFP protein resulting in the appearance of red cells. If a proper donor template to correct the mVenus mutation is delivered into the cells together with the Cas9 nuclease, HDR events can be measured by the appearance of green cells. Consequently the so-called traffic light indicator allows a differentiation between a non-homologous end joining of the DNA break resulting in red cells and a homology-directed repair resulting in green cells with the higher percentage of the green cells indicating that the homology-directed repair is used more frequently by the cell.

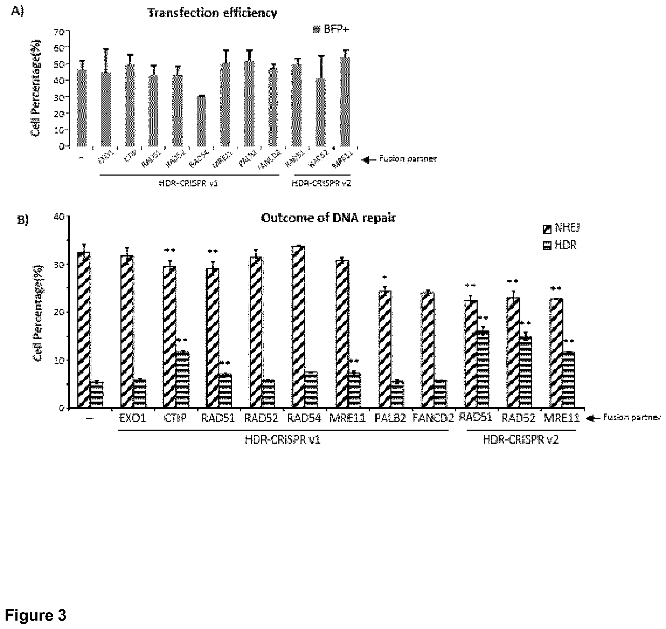

Thereby, the outcome of DNA repair, either NHEJ or HDR, can be easily monitored by flow cytometry in particular by counting the number of cells appearing either red or green. TLR-cells were transfected with a corrective donor DNA and each of the HDR-CRISPR tested in which the Cas9 nuclease is fused to each of the HDR-promoting factors: EXO1, CTIP, RAD51, RAD52, RAD54, MRE11, PALB2, FANCD1. To monitor for transfection efficiency a construct expressing a blue fluorescent protein (BFP) was included in all the samples. Having shown that transfection efficiency was similar in all the samples (**Figure 3A**), the extent of red or green cells 7 days post transfection was measured (**Figure 3B**). While the canonical SpCas9 (without HDR factor) was able to promote HDR in about 5% of the repair events, the extent of HDR was significantly increased for all the HDR-CRISPR v2 and for 3 out 8 HDR-CRISPR v1 analyzed.

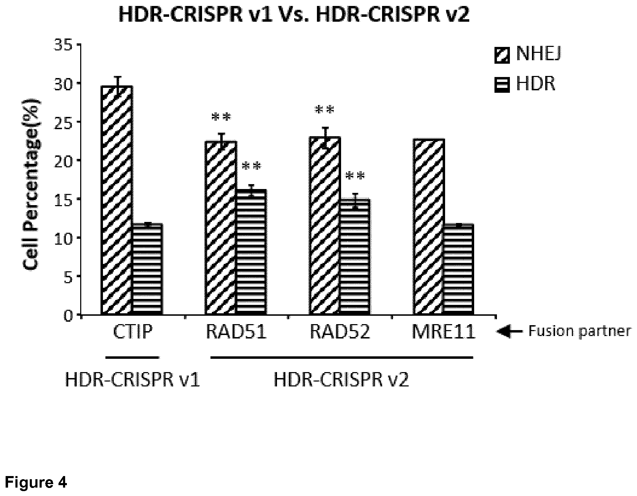

Importantly, the HDR-CRISPR v2 were significantly better in promoting HDR as compared to the previously characterized HDR-CRISPR v1 harboring the SpCas9 fused to the CTIP **(****Figure 4****).**

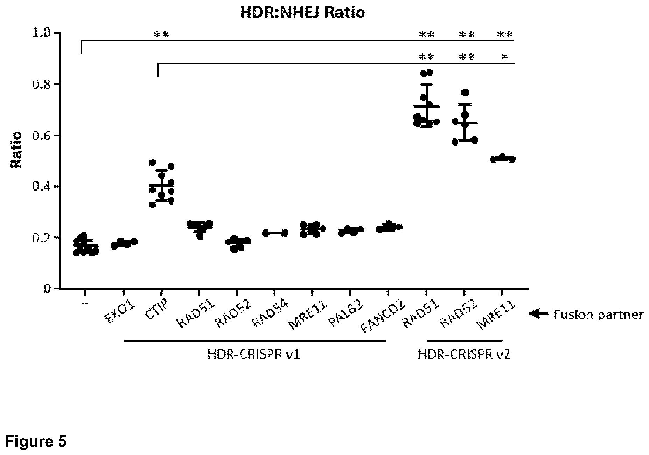

Using our best performing HDR-CRISPR v2 the ratio between the two DNA repair events approached to 1 implying that HDR-mediated DSB repair occurs almost with the same frequency as NHEJ-mediated ones **(****Figure 5****).** In conclusion, a novel platform has been established capable of promoting the repair of a nuclease-induced DSB via HDR. This can be applied to correct disease-causing mutations in the context of primary immunodeficiency or of other genetic defects in general.

### Detailed description of the Figures

**Figure 1****. Schematic of the HDR-CRISPR system.** The different components of HDR-CRISPR v1 and v2 are indicated on the right side as rectangles. The different HDR-promoting proteins or protein domains used in the two version of HDR-CRISPR analyzed are indicated in brackets.
**Figure 2****. Schematic of the reporter system used.** The traffic light reporter system consists of a HEK293T-based cell line harboring integrated in its genome a copy of the indicated reporter construct composed of an mVenus gene with a mutation preventing its fluorescence, fused to an out-of-frame TagRFP gene via a self-cleaving T2A peptide. Upon transfection of the reporter cell line (TLR-cells) with both the HDR-CRISPR and a properly designed donor DNA template for repair of the mVenus sequence, DNA repair events either mediated by non-homologous end-joining (NHEJ) or homologous directed repair (HDR) can be monitor by the occurrence of red or green cells respectively.
**Figure 3****. Efficacy of the HDR-CRISPR system. A)** The histogram shows the transfection efficiency obtained after transfection of the TLR-cells with the mix containing the indicated HDR-CRISPR that included a plasmid expressing a blue fluorescent protein (BFP) to monitor for transfection. The amount of blue cells is measured via flow cytometry three days after transfection (mean ± S.E.M.). **B)** The extent of double strand breaks repaired either via non-homologous end-joining or homologous directed repair is measured via flow cytometry seven days post transfections of the TLR-cells. The extent of cells either showing red or green fluorescence indicative of NHEJ- and HDR-mediated double strand break repair, respectively, are indicated (mean ± S.E.M.). Statistically significant differences, as compared to the SpCas9 without any fusion partner are reported within the graph (two-tailed, paired Student's t-test, *P < 0.05, **P < 0.01).
**Figure 4****. Comparison of HDR-CRISPR systems.** The histogram shows the extent of double strand breaks repaired either via NHEJ or HDR measured as red or green cells respectively 7 days after transfection (mean ± S.E.M.). The data are extracted from those shown in figure 3 to compare side-by-side the efficiencies of the previously published HDR-CRISPR v1 containing a fusion between SpCas9 and CTIP, with our newly established HDR-CRISPR v2. Statistically significant differences, as compared to the indicated HDR-CRISPR are indicated within the graph (two-tailed, paired Student's t-test, **P < 0.01).
**Figure 5****. Extent of DSB repair pathway choice.** The data in figure 3 are shown here as ratio between the DSB repair events occurred either via HDR or NHEJ. Each dot represents a single experiment. Statistically significant differences as compared to the indicated HDR-CRISPR v1 are reported within the graph (two-tailed, paired Student's t-test, *P < 0.05, **P < 0.01).

The amino acid sequences of the highly relevant protein sequences as used in the present invention are provided in the sequence protocol which is part of the present invention. The sequences and the corresponding gene products are listed in the Table below:

| | |
|---|---|
| SEQ ID NO:1 | amino acid linker |
| SEQ ID NO:2 | amino acid linker |
| SEQ ID NO:3 | Cas9 |
| SEQ ID NO:4 | RAD51 |
| SEQ ID NO:5 | RAD52 |
| SEQ ID NO:6 | RAD54 |
| SEQ ID NO:7 | MRE11 |
| SEQ ID NO:8 | PALB2 |
| SEQ ID NO:9 | FANCD2 |
| SEQ ID NO:10 | EXO1 |
| SEQ ID NO:11 | CtIP |
| SEQ ID NO:12 | NLS |
| SEQ ID NO:13 | HA |

### Examples

The HDR factors were isolated from human samples. The sequences as used are shown in the sequence protocol. For isolating the HDR factors RNA from human cells of different origin such as HEK293T, Jurkat, K562 cells was isolated. Via Reverse Transcription PCR (RTPCR) the entire coding region of the HDR factor of interest has been amplified and sequenced. To this end, the primers used for the PCR were selected to bind at the extremities of the coding region from the ATG translation start codon to the stop codon. Moreover, the primers included recognition sites for restriction enzymes which are compatible with the restriction sites present in the plasmid used as suitable vector.

The resulting PCR amplicons were digested with restriction enzymes and cloned in the appropriate vectors. The HDR factors were directly cloned in the HDR-CRISPR v1 or HDR-CRISPR v2 plasmids which are schematically shown in Figure 1. Of course the appropriate HA tag and the NLS sequences were also included.

## Claims

1. Modified Cas9 nuclease comprising a substantial part of a Cas9 nuclease which is required for the biological activity Z and fused thereto at least one of a functional Z protein domain of a HDR factor selected from the group consisting of RAD51, RAD52, and/or MRE11, **characterized in that** it further comprises CtBP interacting protein (CtIP).

2. Modified Cas9 nuclease according to claim 1 **characterized in that** the Cas9 nuclease fragment is derived from Streptococcus pyogenes.

3. Modified Cas9 nuclease according to claim 1 **characterized in that** it comprises at least two HDR factors selected from RAD51, RAD52 and MRE11.

4. Modified Cas9 nuclease according to any of claims 1 to 3 **characterized in that** it comprises a hemagglutination tag.

5. Nucleic acid coding for a modified Cas9 nuclease according to any of claims 1 to 4.

6. Nucleic acid according to claim 5 **characterized in that** it is a DNA sequence and/or **in that** it comprises a promoter derived from CMV.

7. Vector for the transfection of target cells **characterized in that** it comprises a nucleic acid according to claims 5 or 6.

8. Vector according to claim 7 **characterized in that** it is a plasmid.

9. Vector according to claim 7 **characterized in that** it is a vector comprising viral elements.

10. Modified Cas9 nuclease for use in therapeutic editing of nucleic acid sequences in a homology directed repair wherein a modified Cas9 nuclease according to any of claims 1 to 4 and/or a nucleic acid according to claim 5 or 6 and/or a vector according to any of claims 7 to 9 is transferred into target cell.

## Patentansprüche

1. Modifizierte Cas9-Nuklease, umfassend einen wesentlichen Teil einer Cas9-Nuklease, die für die biologische Aktivität erforderlich ist, und daran kondensiert mindestens eine einer funktionellen Proteindomäne eines HDR-Faktors, ausgewählt aus der Gruppe, bestehend aus RAD51, RAD52 und/oder MRE11, **dadurch gekennzeichnet, dass** sie ferner CtBP-Interacting Protein (CtIP) umfasst.

2. Modifizierte Cas9-Nuklease nach Anspruch 1, **dadurch gekennzeichnet, dass** das Cas9-Nukleasefragment von Streptococcus pyogenes abgeleitet ist.

3. Modifizierte Cas9-Nuklease nach Anspruch 1, **dadurch gekennzeichnet, dass** sie mindestens zwei HDR-Faktoren, ausgewählt aus RAD51, RAD52 und MRE11, umfasst.

4. Modifizierte Cas9-Nuklease nach einem der Ansprüche 1 bis 3, **dadurch gekennzeichnet, dass** sie eine Hämagglutinationsmarkierung umfasst.

5. Nukleinsäure, die für eine modifizierte Cas9-Nuklease nach einem der Ansprüche 1 bis 4 kodiert.

6. Nukleinsäure nach Anspruch 5 **dadurch gekennzeichnet, dass** sie eine DNA-Sequenz ist und/oder dadurch, dass sie einen von CMV abgeleiteten Promotor umfasst.

7. Vektor für die Transfektion von Zielzellen, **dadurch gekennzeichnet, dass** er eine Nukleinsäure nach den Ansprüchen 5 oder 6 umfasst.

8. Vektor nach Anspruch 7, **dadurch gekennzeichnet, dass** er ein Plasmid ist.

9. Vektor nach Anspruch 7, **dadurch gekennzeichnet, dass** er ein Vektor ist, der virale Elemente umfasst.

10. Modifizierte Cas9-Nuklease zur Verwendung bei der therapeutischen Editierung von Nukleinsäuresequenzen bei einer Homologie-gesteuerten Reparatur, wobei eine modifizierte Cas9-Nuklease nach einem der Ansprüche 1 bis 4 und/oder eine Nukleinsäure nach Anspruch 5 oder 6 und/oder ein Vektor nach einem der Ansprüche 7 bis 9 in eine Zielzelle überführt wird.

## Revendications

1. Nucléase Cas9 modifiée comprenant une partie substantielle d'une nucléase Cas9 qui est requise pour l'activité biologique et fusionnée à au moins un domaine protéique fonctionnel d'un facteur HDR choisi dans le groupe constitué de RAD51, RAD52, et/ou MRE11, **caractérisée en ce qu'**elle comprend en outre une protéine interagissant avec CtBP (CtIP).

2. Nucléase Cas9 modifiée selon la revendication 1, **caractérisée en ce que** le fragment de nucléase Cas9 est dérivé de Streptococcus pyogenes.

3. Nucléase Cas9 modifiée selon la revendication 1 **caractérisée en ce qu'**elle comprend au moins deux facteurs HDR choisis parmi RAD51, RAD52 et MRE11.

4. Nucléase Cas9 modifiée selon l'une quelconque des revendications 1 à 3 **caractérisée en ce qu'**elle comprend un tag d'hémagglutination.

5. Acide nucléique codant pour une nucléase Cas9 modifiée selon l'une quelconque des revendications 1 à 4.

6. Acide nucléique selon la revendication 5 **caractérisé en ce qu'**il est une séquence d'ADN et/ou **en ce qu'**il comprend un promoteur dérivé du CMV.

7. Vecteur pour la transfection de cellules cibles **caractérisé en ce qu'**il comprend un acide nucléique selon les revendications 5 ou 6.

8. Vecteur selon la revendication 7 **caractérisé en ce qu'**il est un plasmide.

9. Vecteur selon la revendication 7 **caractérisé en ce qu'**il est un vecteur comprenant des éléments viraux.

10. Nucléase Cas9 modifiée pour une utilisation dans l'édition thérapeutique de séquences d'acide nucléique dans une réparation dirigée par homologie dans laquelle une nucléase Cas9 modifiée selon l'une quelconque des revendications 1 à 4 et/ou un acide nucléique selon la revendication 5 ou 6 et/ou un vecteur selon l'une quelconque des revendications 7 à 9 est transférée dans une cellule cible.
